# EUROPEAN PATENT SPECIFICATION

(11) **EP 4 000 556 B1**
(45) Date of publication and mention of the grant of the patent: **15.10.2025**
(21) Application number: 20841277.5
(22) Date of filing: 16.07.2020
(51) Int. Cl.: A61C 8/00, C04B 35/486, A61K 6/84

(54) **DENTAL IMPLANT BODY AND DENTAL IMPLANT BODY MANUFACTURING METHOD**
ZAHNIMPLANTATKÖRPER UND VERFAHREN ZUR HERSTELLUNG DES ZAHNIMPLANTATKÖRPERS
CORPS D'IMPLANT DENTAIRE ET MÉTHODE DE FABRICATION DE CORPS D'IMPLANT DENTAIRE

(30) Priority: 18.07.2019 JP 2019132817
(43) Date of publication of application: 25.05.2022
(73) Proprietor: Orbray Co., Ltd., Tokyo 123-8511 (JP); National Institute of Advanced Industrial Science and Technology, Chiyoda-ku Tokyo 100-8921 (JP)
(72) Inventor: MUTO, Hikaru, Tokyo 123-8511 (JP); FUKUSHIMA, Manabu, Nagoya-shi, Aichi 463-8560 (JP); HYUGA, Hideki, Nagoya-shi, Aichi 463-8560 (JP); YOSHIZAWA, Yuichi, Nagoya-shi, Aichi 463-8560 (JP)
(74) Representative: Dennemeyer & Associates S.A.
(86) International application number: PCT/JP2020/027661
(87) International publication number: WO 2021/010435

(56) References cited:
- WO-A1-2009/072788
- JP-A- 2008 201 636
- JP-A- 2010 018 459
- JP-A- 2010 018 459
- US-A1- 2009 281 633

## Description

### TECHNICAL FIELD

The present invention relates to a dental implant body and a method for manufacturing the dental implant body.

### BACKGROUND ART

For a dental implant body (a tooth root portion; hereinafter merely referred to as an "implant body" as necessary) used for dental implant treatment, not only the strength of the implant body alone but also adherence to jawbone cells for joint between the implant body and a bone are required for resistance to biting force and suppressing in aging deterioration.

Ceramic has been known as the material of such an implant body. Of ceramic, zirconia (ZrO₂) is specifically suitable for the dental implant body because zirconia has properties such as innoxiousness in terms of health in addition to excellent mechanical properties, chemical durability, and thermal resistance.

As one example of such a zirconia dental implant body, Patent Literature 1 has been filed as a patent application, for example. Patent Literature 1 is the application relating to an artificial tooth root zirconia implant member made of partially stabilized zirconia containing yttria. Patent Literature 2 discloses a porous calcium phosphate-based ceramic material having pores penetrating one direction of the ceramic material, and Patent Literature 3 also discloses a porous ceramic material having pores penetrating one direction of the ceramic material, wherein the ceramic material can be zirconia. Patent Literature 4 discloses an implant composed of an aggregate of spherical balls which are connected to each other through sintering.

### CITATION LIST

### PATENT LITERATURE

PATENT LITERATURE 1: JP-A-61-146757
PATENT LITERATURE 2: JP 2010-018459 A
PATENT LITERATURE 3: US 2009/281633 A1
PATENT LITERATURE 4: WO 2009/072788A1

### SUMMARY OF THE INVENTION

### PROBLEMS TO BE SOLVED BY THE INVENTION

However, if a screw type (screw-shaped) implant body is produced from zirconia, chipping or cracking is caused at a thread, leading to the probability that the strength of joint to a jawbone decreases and the implant body drops from the jawbone.

A cylinder type (cylindrical) implant body with no thread is formed with no thread portion, leading to the probability that the implant body is detached from the jawbone.

The present invention has been made in view of the above-described problems, and an object of the present invention is to provide a dental implant body and a dental implant body manufacturing method capable of improving the strength of joint to a jawbone, reducing dropping and detachment from the jawbone, and improving a prognosis.

### SOLUTION TO THE PROBLEMS

The above-described problems are solved by the invention as defined in independent claim 1 of the present invention.

Moreover, the above-described problems are solved by the dental implant body manufacturing method as defined in independent claim 3 of the present invention.

### EFFECTS OF THE INVENTION

According to the dental implant body and the dental implant body manufacturing method of the present invention, degradation of adherence to jawbone cells can be suppressed, and the strength of joint to a jawbone can be improved. With improvement of the joint strength, dropping and detachment of the dental implant body from the jawbone can be reduced, and a prognosis can be improved.

### BRIEF DESCRIPTION OF THE DRAWINGS

[Fig. 1] Fig. 1 shows a SEM observation image of an example of a dental implant body according to the present invention, the observation image showing a section in a direction perpendicular to the direction of formation of blind/continuous holes.
[Fig. 2] Fig. 2 shows a SEM observation image of the example of the dental implant body according to the present invention, the observation image showing a section in the direction of formation of the blind/continuous holes.
[Fig. 3] Fig. 3 shows schematic views of the steps of freezing a gel body in the method for manufacturing the dental implant body according to the present invention.
[Fig. 4] Fig. 4 shows a sectional view schematically showing a ceramics sintered body forming the dental implant body according to the present invention.
[Fig. 5] Fig. 5 shows a sectional view schematically showing a variation of the ceramics sintered body forming the dental implant body according to the present invention.

### DESCRIPTION OF THE EMBODIMENTS

A first feature of the present embodiment is a dental implant body including a ceramics sintered body, the ceramics sintered body being a porous body having blind/continuous holes formed from a surface of the ceramics sintered body and walls formed by the blind/continuous holes.

A second feature of the present embodiment is the dental implant body in which the porosity of the blind/continuous holes is 50 ± 10% and the diameter of the blind/continuous hole is equal to or greater than 50 µm and equal to or smaller than 190 µm.

A third feature of the present embodiment is the method for manufacturing a dental implant body, the method including producing slurry by dispersing ceramic powder in gelatable liquid, producing a gel body by gelating the slurry, freezing the produced gel body, and drying and sintering the gel body to form a porous body including a ceramics sintered body, having blind/continuous holes formed from a surface of the ceramics sintered body, and having walls formed by the blind/continuous holes, thereby forming the dental implant body.

A fourth feature of the present embodiment is the method for manufacturing the dental implant, in which the concentration of the ceramic powder dispersed in the gelatable liquid is set to equal to or higher than 5% and equal to or lower than 65%, the gel body is frozen at a range of equal to or higher than -40°C and equal to or lower than -10°C, and the porous body is formed such that the porosity of the blind/continuous holes is 50 ± 10% and the diameter of the blind/continuous hole is equal to or greater than 50 µm and equal to or smaller than 190 µm.

According to these configuration and manufacturing method, degradation of adherence to jawbone cells can be suppressed or prevented, and the strength of joint to a jawbone can be improved. With improvement of the joint strength, dropping and detachment of the dental implant body from the jawbone can be reduced or prevented, and a prognosis (a progress after dental implant treatment) can be also improved.

Note that in the present invention, the blind/continuous hole indicates a pore formed to appear at a surface of a dental implant body. Further, the continuous hole indicates a pore penetrating a dental implant body from a front surface to a back surface as another surface. On the other hand, the blind hole indicates a pore not penetrating a dental implant body to a back surface.

A fifth feature of the present embodiment is the dental implant body in which the ceramics sintered body is made of zirconia.

A sixth feature of the present embodiment is the method for manufacturing the dental implant body, in which the ceramics sintered body is made of zirconia.

According to these configuration and manufacturing method, zirconia is used for the ceramics sintered body. Thus, the zirconia ceramics sintered body is suitable for the dental implant body because such a ceramics sintered body has properties such as chemical durability, thermal resistance, and innoxiousness in terms of health. Further, favorable mechanical properties (mechanical strength and processability) are obtained.

A seventh feature of the present embodiment is the dental implant body in which the blind/continuous holes are formed in a certain direction and the walls are densely formed.

An eighth feature of the present embodiment is the method for manufacturing the dental implant body, in which ice crystals are grown in a certain direction in the gel body by freezing of the gel body, and accordingly, the blind/continuous holes are formed in the certain direction and the walls are densely formed.

According to these configuration and manufacturing method, the direction of formation of the blind/continuous holes is one direction, and therefore, the walls can be more densely formed. Thus, the dental implant body can be obtained, which has high mechanical properties suitable for dental use while having the blind/continuous holes.

Note that in the present invention, the term "densely" includes not only a wall with no holes, micropores, or nanopores, but also a wall with a density of equal to or higher than 99%.

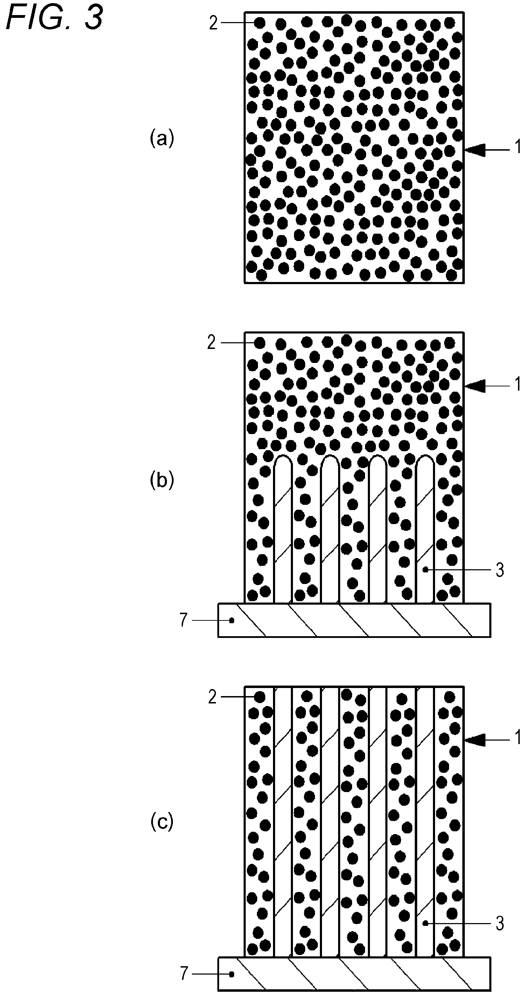

Hereinafter, the method for manufacturing a dental implant body according to the present embodiment will be described in detail with reference to Figs. 3 to 5, and the dental implant body obtained by such a manufacturing method will be described as needed. As the manufacturing method according to the embodiment of the present invention, gelatable polymeric liquid is first prepared, and ceramic powder is dispersed in the liquid at a powder concentration of equal to or higher than 5% and equal to or lower than 65%. Accordingly, slurry is produced.

The ceramic powder dispersed in the liquid is made of zirconia (ZrO₂) and is granulated, and the average particle size of the ceramic powder is set to a range of 0.01 µm (10 nm) to 0.08 µm (80 nm). If the average particle size is smaller than 0.01 µm, it is not preferred because the ceramic powder is difficult to be handled and workability is degraded. On the other hand, if the average particle size exceeds 0.08 µm, it is not preferred because the ceramic powder is easily precipitated in the slurry and the slurry cannot be stably obtained.

The water content of the slurry is set to 35 wt% to 95 wt%. If the water content is lower than 35 wt%, the ceramic powder is aggregated and easily precipitated and a stable dispersion state is difficult to be held. On the other hand, if the water content exceeds 95 wt%, the density of a ceramic molded body after water has been sublimed into ice crystals is extremely low and the ceramic molded body cannot satisfy a strength as the dental implant body.

Next, the produced slurry is gelated, and a gel body is produced. Gelation indicates that the slurry in which the ceramic powder is dispersed is solidified, and the gel body is formed with a cylindrical outer shape. Fig. 3(a) schematically shows a gel body 1. Black circles in the gel body 1 indicate dispersed ceramic powder 2.

Next, the produced gel body 1 is frozen at a range of equal to or higher than -40°C and equal to or lower than -10°C. The gel body 1 is frozen in such a manner that a bottom surface of the gel body 1 contacts a copper or aluminum freezing plate 7 as shown in Fig. 3(b) and the gel body 1 is, by heat transfer, cooled in a certain direction (an upward direction in Fig. 3(b)) from the bottom surface to the other side. Note that Fig. 3(b) is a sectional view of the gel body 1 cut along an optional section and the section of the gel body 1 in which the ceramic powder 2 is dispersed is shown without hatching for the sake of viewability.

Since the gel body 1 is frozen in the certain direction from the bottom surface contacting the freezing plate 7, multiple ice crystals 3 frozen in a frost column shape from water without the ceramic powder 2 being dispersed are formed in the certain direction in the gel body 1 as shown from Fig. 3(b) to Fig. 3(c). Note that Fig. 3(c) is also a sectional view of the gel body 1 cut along an optional section as in Fig. 3(b) and the section of the gel body 1 in which the ceramic powder 2 is dispersed is shown without hatching for the sake of viewability. In Figs. 3(b) and 3(c), hatched portions indicate the ice crystals 3.

Meanwhile, the ceramic powder 2 is unevenly distributed in regions of the gel body 1 other than the ice crystals 3, as shown in Figs. 3(b) and 3(c). Water expands due to freezing, and for this reason, the regions in which the ceramic powder is unevenly distributed are pushed and compressed by the ice crystals 3. Due to such compression, the regions in which the ceramic powder is unevenly distributed are densified, and later-described walls are formed accordingly. Note that the thickness (the dimension in the upper-lower direction in Fig. 3) of the gel body 1 shown in Fig. 3 is set to 1.2 µm to 130 µm.

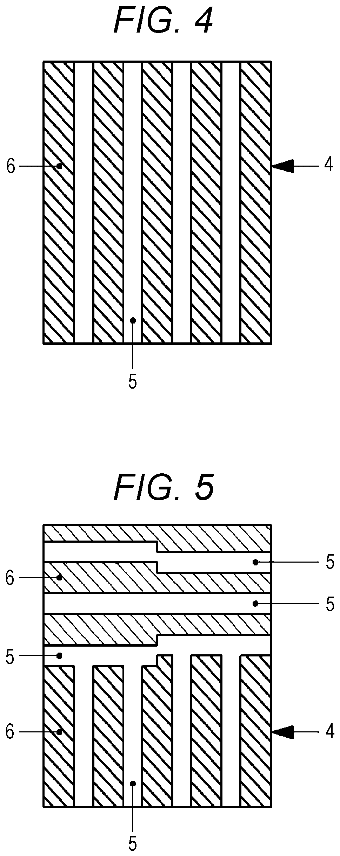

Next, the frozen gel body is dried in atmosphere, and the ice crystals 3 are sublimed to obtain a ceramic molded body. Thereafter, the ceramic molded body is sintered to form a ceramics sintered body 4 shown in Fig. 4. Note that Fig. 4 is a sectional view of the ceramics sintered body 4 cut along an optional section. A sintering method is atmospheric sintering, a heating temperature is 2°C/min to 10°C/min, a sintering temperature is 1300°C to 1500°C, atmosphere is atmospheric air, a pressure is an ordinary pressure, and a sintering time is one hour to four hours.

The ice crystals 3 are sublimed as described above, and are sintered thereafter. Accordingly, blind/continuous holes 5 are formed from a surface of the ceramics sintered body 4 as shown in Fig. 4, and a porous body in which a wall 6 is formed between adjacent ones of the blind/continuous holes 5 can be formed. Fig. 4 shows, as one example, the ceramics sintered body 4 in which only the continuous holes 5 are formed as pores and the wall 6 is formed between adjacent ones of the continuous holes 5.

The blind/continuous hole indicates a pore formed to appear at a surface of a dental implant body including a ceramics sintered body. Further, the continuous hole indicates, as shown in Fig. 4, a pore penetrating a dental implant body from a front surface to a back surface as another surface. On the other hand, the blind hole indicates a pore not penetrating a dental implant body to a back surface.

Since the dental implant body is formed of the above-described porous body and the blind/continuous holes are formed from the surface, jawbone cell tissues can smoothly enter the blind/continuous holes when the dental implant body is fixed to a jawbone. Thus, degradation of adhesion of the implant body to jawbone cells can be suppressed without the need for forming a thread at the implant body, and the strength of joint to the jawbone can be improved. With improvement of the joint strength, dropping and detachment of the dental implant body from the jawbone can be reduced, and a prognosis (a progress after dental implant treatment) can be also improved. Moreover, there are no concerns about chipping and cracking due to thread formation.

Further, in the present embodiment, the porosity of the blind/continuous holes is set to 50 ± 10%, and the diameter of the blind/continuous hole is set to equal to or greater than 50 µm and equal to or smaller than 190 µm. That is, in the present embodiment, all of the diameters of the blind/continuous holes formed from the surface of the ceramics sintered body are within a range of equal to or greater than 50 µm and equal to or smaller than 190 µm. The numerical values of the porosity and the diameter are set as described above, and these values are achieved simultaneously in the single porous body. Thus, the jawbone cell tissues can enter all of the blind/continuous holes. Consequently, degradation of adhesion of the implant body to the jawbone cells can be prevented without the need for forming the thread at the implant body, and the strength of joint to the jawbone can be further improved. With further improvement of the joint strength, dropping and detachment of the dental implant body from the jawbone can be prevented, and the prognosis can be also improved.

The porosity is 50% as a center value, and changes within a range of ±10%. It has found that if the porosity exceeds 50% + 10% (i.e., 60%), the implant body formed of the porous body does not satisfy a strength for dental use for which resistance to biting force in daily diet and suppression in aging deterioration due to biting are required. On the other hand, it has found that if the porosity is less than 50% - 10% (i.e., 40%), the number of jawbone cell tissues which can enter the blind/continuous holes decreases in association with a decrease in the porosity and adhesion of the implant body to the jawbone cells is degraded.

Further, it has found that if the diameter of the blind/continuous hole exceeds 190 µm, the diameter is too expanded, the growth rate of the jawbone cells in the blind/continuous hole decreases, and the strength of joint between the implant body and the jawbone decreases. On the other hand, it has found that if the diameter of the blind/continuous hole is smaller than 50 µm, the jawbone cell tissues are difficult to enter the blind/continuous holes, adhesion of the implant body to the jawbone cells is degraded, and the strength of joint between the implant body and the jawbone decreases.

As described above, it has confirmed that in the dental implant body formed of the porous body, both of a blind/continuous hole porosity of 50 ± 10% and a blind/continuous hole diameter of equal to or greater than 50 µm and equal to or smaller than 190 µm are essential for preventing degradation of adhesion of the dental implant body to the jawbone cells and preventing dropping and detachment of the dental implant body from the jawbone.

The porosity can be measured by a mercury intrusion technique using a mercury porosimeter. Moreover, the diameter of the blind/continuous hole is measured using a scanning electron microscope (SEM) observation image.

In the present embodiment, it has found that as the conditions for achieving both of a blind/continuous hole porosity of 50 ± 10% and a blind/continuous hole diameter of equal to or greater than 50 µm and equal to or smaller than 190 µm, it is essential to set the concentration of the ceramic powder dispersed in the gelatable liquid to equal to or higher than 5% and equal to or lower than 65% and set a gel body freezing temperature to a range of equal to or higher than -40°C and equal to or lower than -10°C. If either one of the concentration of the ceramic powder 2 or the freezing temperature of the gel body 1 falls outside the concentration or temperature range, both of the porosity and the diameter cannot be set within desired ranges.

Further, if the concentration of the ceramic powder 2 is lower than 5%, the strength of the dental implant body cannot be satisfied due to an extremely-low density of the ceramic molded body. If the concentration exceeds 65%, the ceramic powder is aggregated and easily precipitated, and the stable dispersion state is difficult to be held.

If the gel body freezing temperature reaches lower than -40°C, the entire gel body is frozen before the ice crystals are formed in the frost column shape, and the ceramic powder cannot be unevenly distributed in the gel body regions other than the ice crystals. In addition, if the freezing temperature exceeds -10°C, the ice crystals are difficult to be formed in the gel body.

Further, in the present embodiment, according to the porous body used for the dental implant body and the method for manufacturing such a porous body, the walls 6 are more preferably densely formed in such a manner that the gel body 1 is frozen such that the ice crystals 3 are grown in the certain direction in the gel body 1 as shown in Fig. 3 and the blind/continuous holes 5 are formed in the certain direction as shown in Fig. 4. The term "densely" includes not only a wall with no holes, micropores, or nanopores, but also a wall with a density of equal to or higher than 99%. A reason why the dense wall is not limited to one with a density of 100% is that in addition to the frost-columnar ice crystals, ice having an extremely-smaller diameter than that of the ice crystal is formed in the walls in the gel body at the above-described freezing step in some cases. Such ice is also sublimed upon drying of the gel body, and therefore, fine holes or pores are formed after sublimation of the ice and holes, micropores, or nanopores are formed in the walls in some cases.

Note that the certain direction in which the blind/continuous holes are formed indicates one formation direction such as the continuous holes 5 of Fig. 4 formed from the front surface to the back surface (from up to down in Fig. 4) of the ceramics sintered body 4.

It is necessary for formation of the blind/continuous holes in the certain direction to form the ice crystals in the certain direction in the gel body. As a result of study conducted by the applicant of the present application, the condition necessary for formation of the ice crystals in the certain direction is that the gel body is, by heat transfer, gradually frozen from one location to the other location along the certain direction at a range of equal to or higher than -40°C and equal to or lower than -10°C.

In Figs. 3(b) and 3(c), the surface of the gel body 1 contacting the freezing plate 7 is one location from which freezing is started, and the gel body 1 is, by heat transfer, gradually frozen to the upper surface as the other location along the certain direction (the upward direction in Figs. 3(b) and 3(c)).

The direction of formation of the blind/continuous holes is one direction so that the ceramic powder can be unevenly distributed with regularity and the ice crystals can uniformly apply compression force to the entire surfaces of the walls. Thus, the walls can be more densely formed. Thus, the dental implant body can be obtained, which has high mechanical properties (mechanical strength and processability) suitable for dental use while having the blind/continuous holes. Consequently, in addition to setting of the porosity and diameter of the blind/continuous holes, the direction of formation of the blind/continuous holes is, for the dental implant body, more preferably set to one direction.

The blind/continuous holes may be formed in the right-left direction, an oblique direction, or a combination thereof in the ceramics sintered body. Fig. 5 shows a variation example where multiple continuous holes 5 are formed from side surfaces in the horizontal direction as viewed in the figure at the upper half of the ceramics sintered body 4 and multiple continuous holes 5 are formed in the vertical direction as viewed in the figure at the lower half as in Fig. 4. Note that Fig. 5 is a sectional view of the ceramics sintered body 4 cut along an optional section. Of the multiple continuous holes 5 formed in the horizontal direction, the lowermost continuous hole 5 communicates with the continuous holes 5 formed in the vertical direction.

For the ceramics sintered body 4 of Fig. 5, the freezing plate 7 is arranged on the bottom surface to form the continuous holes 5 in the vertical direction, and a freezing agent is arranged on the entire circumference of the side surfaces of the upper half of the gel body to form the multiple continuous holes 5 from the side surfaces to the center of the gel body. Thus, in some cases, the continuous holes communicate with each other at shifted positions as in the uppermost and lowermost ones of the multiple continuous holes 5 in the horizontal direction as shown in Fig. 5. In other cases, the continuous holes communicate with each other at coincident positions as in the continuous hole 5 formed in the middle.

Further, in the present embodiment, zirconia is used as the ceramic powder which is a raw material, and therefore, the ceramics sintered body is made of zirconia. The zirconia ceramics sintered body is suitable for the dental implant body because such a ceramics sintered body has properties such as chemical durability, thermal resistance, and innoxiousness in terms of health. Further, favorable mechanical properties (mechanical strength and processability) are obtained.

Considering application of zirconia to the dental implant body as a biological reinforcement member, yttria (yttrium oxide: Y₂O₃) containing zirconia is more preferred for ensuring higher mechanical strength. Specific examples include 2Y zirconia (2 mol% yttria containing zirconia), 2.5Y zirconia (2.5 mol% yttria containing zirconia), 3Y zirconia (3 mol% yttria containing zirconia), and 8Y zirconia (8 mol% yttria containing zirconia).

The color of the implant body obtained as described above is white, and the Vickers hardness of the implant body is 10 GPa in one direction in which the blind/continuous holes are formed and is 3.5 GPa in other directions. The implant body can be formed into a one-piece type or a two-piece type with another abutment (support).

An example according to the present invention will be described below, but the present invention is not limited only to the following example.

### Example

Hereinafter, the method for manufacturing a dental implant body according to the present example will be described. First, gelatable polymeric liquid was prepared, and granulated zirconia powder was dispersed in the liquid at a powder concentration of 35%. Accordingly, slurry was produced. In the present example, polycrystalline zirconia containing no yttria, sintered for two hours at a temperature range of 1350°C to 1450°C, and having a density of 99% was used as a dense body.

Next, the produced slurry was gelated, and a gel body was produced. A copper freezing plate 7 contacted a bottom surface of the gel body as in Fig. 3(b), and the gel body was, by heat transfer, cooled in a certain direction from the bottom surface to the other side and was frozen at -20°C. Accordingly, multiple ice crystals were formed in the certain direction as in Fig. 3(c).

Next, the frozen gel body was dried in atmosphere, and the ice crystals were sublimed to obtain a ceramic molded body. The ceramic molded body was sintered to form a ceramics sintered body. A sintering method was atmospheric sintering, a heating temperature was 2°C/min, a sintering temperature was 1350°C, atmosphere was atmospheric air, a pressure was an ordinary pressure, and a sintering time was two hours.

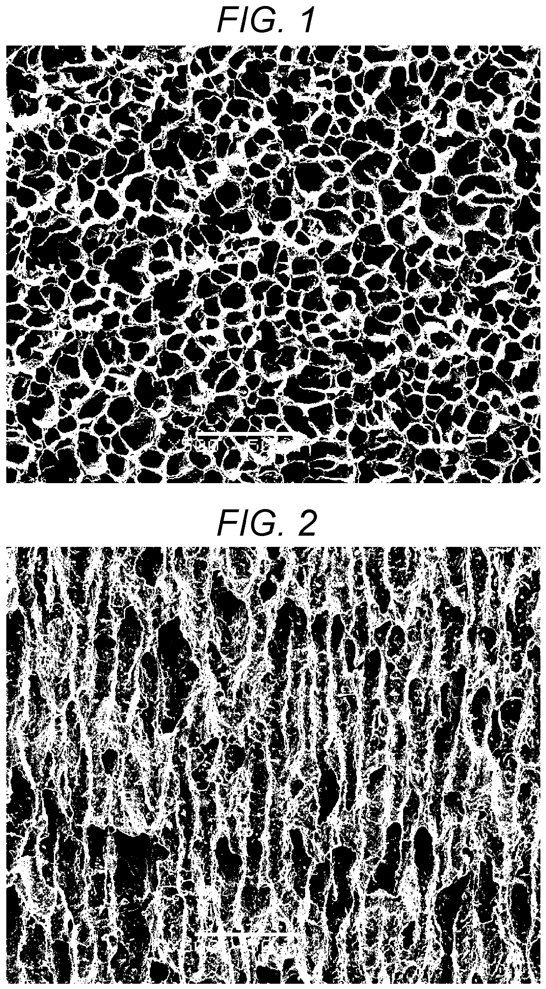

SEM observation images of the obtained ceramics sintered body are shown in Figs. 1 and 2. Fig. 1 shows the observation image in a section perpendicular to the direction of formation of the blind/continuous holes, and Fig. 2 shows the observation image in a section in the direction of formation of the blind/continuous holes. From Fig. 1, it has confirmed that the blind/continuous holes are formed from a surface of the ceramics sintered body. From Fig. 2, it has also confirmed that the blind/continuous holes are formed in the certain direction (the upper-lower direction in Fig. 2). Moreover, it has also confirmed that a wall density is 99% and walls are densely formed.

As a result of measurement of the porosity of the blind/continuous holes of the ceramics sintered body by a mercury intrusion technique using a mercury porosimeter, it has found that the porosity is 60%. As a result of observation of the diameter of the blind/continuous hole by means of the SEM observation images, it has also found that all diameters observed at multiple random locations of the SEM observation images are within a range of equal to or greater than 50 µm and equal to or smaller than 190 µm.

Jawbone cell tissues of subjects randomly selected in terms of sex and age adhered to a surface of the ceramics sintered body, and entrance into the blind/continuous holes was observed. Further, it has confirmed that the jawbone cell tissues having entered the blind/continuous holes are held adhered to the surface of the ceramics sintered body without detachment.

### LIST OF REFERENCE NUMERALS

- 1: Gel Body
- 2: Ceramic Powder
- 3: Ice Crystal
- 4: Ceramics Sintered Body
- 5: Blind/Continuous holes
- 6: Wall
- 7: Freezing Plate

## Claims

1. A dental implant body comprising:
a ceramics sintered body (4),
wherein the ceramics sintered body (4) is a porous body having a blind hole or a continuous hole formed from a surface of the ceramics sintered body and a wall (6) formed by the blind hole or a continuous hole,
wherein the continuous hole is a pore penetrating the dental implant body from a front surface to a back surface as another surface, and the blind hole is a pore not penetrating the dental implant body to a back surface,
the ceramics sintered body (4) is made of zirconia, and
a porosity of the ceramics sintered body (4), measured by a mercury intrusion technique using a mercury porosimeter, is 50 ± 10%, wherein
the blind hole or the continuous hole is formed in a certain direction, and the wall (6) is densely formed.

2. The dental implant body according to claim 1, wherein
a diameter of the blind hole or the continuous hole, measured using a scanning electron microscope, is equal to or greater than 50 µm and equal to or smaller than 190 µm.

3. A method for manufacturing a dental implant body, comprising:
producing slurry by dispersing ceramic powder (2) in gelatable liquid;
producing a gel body (1) by gelating the slurry;
freezing the produced gel body (1); and
drying and sintering the gel body (1) to form a porous body including a ceramics sintered body (4), having a blind hole or a continuous hole formed from a surface of the ceramics sintered body, and having a wall (6) formed by the blind hole or the continuous hole, thereby forming the dental implant body,
wherein the continuous hole is a pore penetrating the dental implant body from a front surface to a back surface as another surface, and the blind hole is a pore not penetrating the dental implant body to a back surface,
the ceramics sintered body (4) is made of zirconia, and
a porosity of the ceramics sintered body (4), measured by a mercury intrusion technique using a mercury porosimeter, is 50 ± 10%, wherein
an ice crystal is grown in a certain direction in the gel body by freezing of the gel body, and accordingly, the blind hole or the continuous hole is formed in the certain direction and the wall (6) is densely formed.

4. The method for manufacturing the dental implant body according to claim 3, wherein
a concentration of the ceramic powder (2) dispersed in the gelatable liquid is set to equal to or higher than 5% and equal to or lower than 65%,
the gel body (1) is frozen at a range of equal to or higher than -40°C and equal to or lower than -10°C, and
the porous body is formed such that a diameter of the blind hole or the continuous hole, measured using a scanning electron microscope, is equal to or greater than 50 µm and equal to or smaller than 190 µm.

## Patentansprüche

1. Zahnimplantatkörper, umfassend:
einen gesinterten Keramikkörper (4),
wobei der gesinterte Keramikkörper (4) ein poröser Körper ist, der ein blindes Loch oder ein kontinuierliches Loch, das aus einer Fläche des gesinterten Keramikkörpers ausgebildet ist, und eine Wand (6), die von dem blinden Loch oder einem kontinuierlichen Loch ausgebildet ist, aufweist,
wobei das kontinuierliche Loch eine Pore ist, die den Zahnimplantatkörper von einer Vorderfläche zu einer Rückfläche als eine andere Fläche durchdringt, und das blinde Loch eine Pore ist, die den Zahnimplantatkörper nicht zu einer Rückfläche durchdringt,
der gesinterte Keramikkörper (4) aus Zirkonium gefertigt ist, und
eine Porosität des gesinterten Keramikkörpers (4), gemessen durch eine Quecksilberintrusionstechnik unter Verwendung eines Quecksilberporosimeters 50 ± 10 % beträgt, wobei
das blinde Loch oder das kontinuierliche Loch in eine bestimmte Richtung ausgebildet ist, und die Wand (6) dicht ausgebildet ist.

2. Zahnimplantatkörper nach Anspruch 1, wobei
ein Durchmesser des blinden Lochs oder des kontinuierlichen Lochs, gemessen unter Verwendung eines Rasterelektronenmikroskops, gleich oder größer als 50 µm und gleich oder kleiner als 190 µm ist.

3. Verfahren zum Herstellen eines Zahnimplantatkörpers, umfassend:
Produzieren von Schlamm durch Dispergieren von Keramikpulver (2) in gelierfähiger Flüssigkeit;
Produzieren eines Gelkörpers (1) durch Gelieren des Schlamms;
Einfrieren des produzierten Gelkörpers (1); und
Trocknen und Sintern des Gelkörpers (1), um einen porösen Körper auszubilden, der einen gesinterten Keramikkörper (4) beinhaltet, der ein blindes Loch oder ein kontinuierliches Loch aufweist, das aus einer Fläche des gesinterten Keramikkörpers ausgebildet ist, und der eine Wand (6) aufweist, die von dem blinden Loch oder dem kontinuierlichen Loch ausgebildet ist, wodurch der Zahnimplantatkörper ausgebildet wird,
wobei das kontinuierliche Loch eine Pore ist, die den Zahnimplantatkörper von einer Vorderfläche zu einer Rückfläche als eine andere Fläche durchdringt, und das blinde Loch eine Pore ist, die den Zahnimplantatkörper nicht zu einer Rückfläche durchdringt,
der gesinterte Keramikkörper (4) aus Zirkonium gefertigt ist, und
eine Porosität des gesinterten Keramikkörpers (4), gemessen durch eine Quecksilberintrusionstechnik unter Verwendung eines Quecksilberporosimeters 50 ± 10 % beträgt, wobei
ein Eiskristall in einer bestimmten Richtung in dem Gelkörper durch Einfrieren des Gelkörpers gezüchtet wird, und dementsprechend das blinde Loch oder das kontinuierliche Loch in der bestimmten Richtung ausgebildet ist und die Wand (6) dicht ausgebildet ist.

4. Verfahren zum Herstellen des Zahnimplantatkörpers nach Anspruch 3, wobei
eine Konzentration des Keramikpulvers (2), das in der gelierfähigen Flüssigkeit dispergiert ist, so festgelegt wird, dass sie gleich oder größer als 5 % und gleich oder kleiner als 65 % ist,
der Gelkörper (1) in einem Bereich von gleich oder höher als -40 °C und gleich oder kleiner als -10 °eingefroren ist, und
der poröse Körper so ausgebildet ist, dass ein Durchmesser des blinden Lochs oder des kontinuierlichen Lochs, gemessen unter Verwendung eines Rasterelektronenmikroskops, gleich oder größer als 50 µm und gleich oder kleiner als 190 µm ist.

## Revendications

1. Corps d'implant dentaire comprenant :
un corps fritté en céramique (4),
dans lequel le corps fritté en céramique (4) est un corps poreux ayant un trou borgne ou un trou continu formé à partir d'une surface du corps fritté en céramique et d'une paroi (6) formée par le trou borgne ou un trou continu,
dans lequel le trou continu est un pore pénétrant le corps d'implant dentaire d'une surface avant vers une surface arrière en tant qu'autre surface, et le trou borgne est un pore ne pénétrant pas le corps d'implant dentaire vers une surface arrière,
le corps fritté en céramique (4) est fait de zircone, et
une porosité du corps fritté en céramique (4), mesurée par une technique d'intrusion de mercure à l'aide d'un porosimètre à mercure, est de 50 ± 10 %, dans lequel
le trou borgne ou le trou continu est formé dans une certaine direction, et la paroi (6) est formée de manière dense.

2. Corps d'implant dentaire selon la revendication 1, dans lequel
un diamètre du trou borgne ou du trou continu, mesuré à l'aide d'un microscope électronique à balayage, est égal ou supérieur à 50 µm et égal ou inférieur à 190 µm.

3. Procédé de fabrication d'un corps d'implant dentaire, comprenant :
la production d'une suspension en dispersant une poudre de céramique (2) dans un liquide gélifiable ;
la production d'un corps de gel (1) en gélifiant la suspension ;
la congélation du corps de gel (1) produit ; et
le séchage et le frittage du corps de gel (1) pour former un corps poreux incluant un corps fritté en céramique (4), ayant un trou borgne ou un trou continu formé à partir d'une surface du corps fritté en céramique, et ayant une paroi (6) formée par le trou borgne ou le trou continu, formant ainsi le corps d'implant dentaire,
dans lequel le trou continu est un pore pénétrant le corps d'implant dentaire d'une surface avant vers une surface arrière en tant qu'autre surface, et le trou borgne est un pore ne pénétrant pas le corps d'implant dentaire vers une surface arrière,
le corps fritté en céramique (4) est fait de zircone, et
une porosité du corps fritté en céramique (4), mesurée par une technique d'intrusion de mercure à l'aide d'un porosimètre à mercure, est de 50 ± 10 %, dans lequel
un cristal de glace est développé dans une certaine direction dans le corps de gel par congélation du corps de gel, et en conséquence, le trou borgne ou le trou continu est formé dans la certaine direction et la paroi (6) est formée de manière dense.

4. Procédé de fabrication du corps d'implant dentaire selon la revendication 3, dans lequel
une concentration de la poudre de céramique (2) dispersée dans le liquide gélifiable est définie à une valeur égale ou supérieure à 5 % et égale ou inférieure à 65 %,
le corps de gel (1) est congelé dans une plage égale ou supérieure à -40 °C et égale ou inférieure à -10 °C, et
le corps poreux est formé de telle sorte qu'un diamètre du trou borgne ou du trou continu, mesuré à l'aide d'un microscope électronique à balayage,est égal ou supérieur à 50 µm et égal ou inférieur à 190 µm.
